# EUROPEAN PATENT APPLICATION

(11) **EP 0 984 070 A1**
(43) Date of publication of application: **08.03.2000**
(21) Application number: 98116415.5
(22) Date of filing: 31.08.1998
(51) Int. Cl.: C12Q 1/48, G01N 33/68, G01N 33/50

(54) **Method for identifying substances for the treatment of disorders associated with c-Jun-mediated apoptosis**

(71) Applicant: Boehringer Ingelheim International GmbH, 55216 Ingelheim (DE)
(72) Inventor: Behrens, Axel, Dr., A-1150 Wien (AT); Wagner, Erwin F., Prof. Dr., A-1030 Wien (AT); Sibilia, Maria, Dr., A-1030 Wien (AT)

(57) **Abstract**

Substances for the treatment of disorders associated with c-Jun-mediated apoptosis, e.g. treatment of neurodegenerative diseases, have the ability to inhibit c-Jun N-terminal phosphorylation. Methods for screening such substances are provided.

## Description

The present invention relates to the therapy of diseases caused by excitotoxic apoptosis.

Excitotoxic apoptosis may be caused by excitatory amino acids that induce both acute membrane depolarization and latent cellular toxicity, which often leads to apoptosis in many neurological disorders (26).

Glutamate is an important neurotransmitter, but can also, when present in high concentrations, cause neuronal death. Glutamate neurotoxicity has been associated with a number of clinically relevant neurologic diseases including epilepsy, stroke and Huntington's disease (26).

C-Jun, a major component of the heterodimeric transcription factor AP-1, has been implicated in a number of biological processes such as cell proliferation, cell differentiation and apoptosis (1). An important way of regulating c-Jun and thereby AP-1 activity is achieved by c-Jun N-terminal phosphorylation (JNP) at serines 63 and 73 through the Jun N-terminal kinases (JNKs) (2). Many extracellular stress signals activate JNKs (3), which phosphorylate c-Jun and other substrates such as ATF2, ELK1 and NF-AT4 (4). N-terminally phosphorylated c-Jun is proposed to recruit the coactivator proteins CBP and p300 to target gene promoters resulting in increased gene transcription (5).

Fetuses lacking *c-jun* die at mid-gestation (6, 7), exhibit impaired hepatogenesis (6) and primary *c-jun*-/-fibroblasts have a severe proliferation defect and undergo premature senescence in vitro (7). *C-jun*-/-fetal livers are characterized by increased apoptosis of both hepatoblasts and hematopoetic cells (6). JNP is thought to be required for the anti-apoptotic function of c-Jun, since mice lacking the JNK kinase SEK1 exhibit a liver defect similar to *c-jun*-/- fetuses (8). On the other hand, c-Jun and JNP seem to be involved in inducing apoptosis in neurons since overexpression of dominant negative alleles of *c-jun* blocks apoptosis in cultured neurons (9).

The JNK signaling pathway and JNP have been implicated in a variety of biological processes.

Recent studies indicate that glutamate toxicity may involve the c-Jun amino-terminal kinase (JNK) group of mitogen-activated protein (MAP) kinases (9,11,18). This was confirmed by showing that mice lacking the brain-specific JNK3 isoenzyme are protected from excitotoxic neuronal apoptosis induced by the glutamate-receptor agonist kainate (10). Systemic administration of kainate, a glutamate analogue that selectively binds to and activates the AMPA/kainate subclass of glutamate receptors. Based on their findings, these authors suggest JNK3 as a therapeutic target. However, the substrates of JNK3 during neuronal apoptosis are not known.

These authors failed to demonstrate that JNK activity is required cell autonomously for the execution of apoptosis in neurons. It was shown that JNK3 deficient mice exhibit a decreased level of c-Jun N-terminal phosphorylation, but no causal relationship between c-Jun activation and neuronal apoptosis has been demonstrated.

It is known that JNK3 phosphorylates a number of substrates, but it has not been shown which of the numerous JNK3 targets is required for neuronal apoptosis.

Furthermore, the role of c-Jun N-terminal phosphorylation for apoptosis in other cell types, e.g. T-cells, which may have an implication in immunological disorders, is unclear.

It has been an object of the present invention to elucidate the mechanisms underlying JNK-mediated cell death, in particular neuronal apoptosis, and to identify the component(s) in the signal transduction pathway downstream of JNK, in order to make these components available as targets for highly specific inhibition.

It was a further object of the invention to provide improved therapies for the treatment of diseases associated with c-Jun mediated cell death, in particular neurodegenerative diseases and/or immune diseases. Furthermore, it was an object of the invention to identify the death effector genes activated during excitotoxic apoptosis.

To solve the problem underlying the invention, it was started from the hypothesis that c-Jun may be the essential substrate required for JNK-mediated apoptosis, and this hypothesis was tested.

To test the function of c-Jun N-terminal phosphorylation for apoptosis of neurons and other cells, mice and cells carrying a c-Jun allele which lack the JNK phosphoacceptor serines were used. The biological activities of the AP-1 transcription factor c-Jun have been thought to be dependent on N-terminal phosphorylation at serine 63 and 73. Fetuses lacking *c-jun* die during embryogenesis and exhibit liver defects. It was therefore particularly surprising that c-Jun N-terminal phosphorylation is not required for embryonic development, but essential for neuronal and T-cell apoptosis. Furthermore, it has been shown that c-Jun N-terminal phosphorylation is required in neurons specifically for the apoptotic process. It has also been shown that the death effector gene fasL is under direct control of c-Jun N-terminal phosphorylation, which suggests that c-Jun-mediated neuronal apoptosis is, *inter alia*, effected by fasL.

Based on these findings, the present invention provides methods for identifying drugs for the treatment of disorders associated with c-Jun-mediated apoptosis. The methods are characterized in that substances are tested for their ability to inhibit c-Jun N-terminal phosphorylation.

In a preferred embodiment of the invention, a cell-free assay is employed.

The principle of such assay is to inhibit the physical interaction of JNK, preferably JNK3, with its substrate, c-Jun. Since the JNK binding domains for c-Jun have a high degree of homology and both the JNK binding domain on c-Jun, the δ-domain, and the c-Jun binding domain on JNK2 (42) have been identified, the biochemical tools for the assay are easily available.

The assay is conducted by incubating the JNK binding domain for c-Jun and the c-Jun binding domain on JNK, one of them being fused to a reporter gene product and the other one being immobilized on a suitable carrier, e.g. micro titer plates, with the test substance and by determining the effect of the substance on the interaction of the two proteins by measuring the reporter activity. If a substance is an inhibitor of JNK/c-Jun interaction, the reporter signal which results from binding of the two domains to one another, is decreased or abolished. Alternatively, larger proteins containing the binding domains may be used, e.g. the full length proteins, or one of the interaction partner as a full-length protein and the other as the binding domain only, preferably the c-Jun N-terminal peptide and the full length JNK.

In a first embodiment of such an assay, a c-Jun kinase, preferably JNK3, or the c-Jun binding domain of the c-Jun kinase, is recombinantly produced by conventional methods, by expressing the cDNA encoding JNK or the binding domain (44) in a suitable host. Next, the c-Jun terminus, encompassing approximately the N-terminal 100-200 amino acids, is fused to a reporter gene, preferably GFP. This fusion protein can be obtained by recombinant DNA technology as follows: the DNA sequence encoding the c-Jun N-terminus (45) is ligated to the reporter gene sequence and inserted into a suitable expression vector, e.g. in a bacterial plasmid, and expressed in the host.

Alternatively, the reporter protein can be fused in an analogous manner to the JNK protein or its c-Jun binding domain, respectively.

Alternatively, one of the interaction partners may be labeled by any other detectable marker, preferably a marker producing a signal that can be easily measured in a high throughput screen, in which the unlabeled interaction molecule is immobilized, e.g. on a microtiter plate surface or on beads. Examples for suitable labels are radioactive labels or commercially available fluorescent markers suitable for labeling proteins or peptides. Furthermore, the assay may be carried out as a proximity assay using commercially availabe ingredients. Alternatively, one of the interaction partners may be conjugated to an enzyme, e.g. alkaline phosphatase, which allows for monitoring the interaction by determining the enzymatic activity.

To verify the specificity of the substances which have shown an inhibiting effect on c-Jun/JNK interaction, additional known substrates of c-Jun, e.g. ATF2, NF-AT4 (4) are tested for their interaction with JNK in the presence or the absence of the substance. Specific inhibitors abolish c-Jun binding to JNK, but not the binding of other substrates to JNK.

In an alternative embodiment of the invention, a screening method is provided which is based on mammalian cells, preferably human cells, in which the inhibition of c-Jun N-terminal phosphorylation can be monitored.

One way of monitoring c-Jun N-terminal phosphorylation is determining the expression of a reporter gene under the control of a regulatory element of a c-Jun target gene, e.g. fasL, which is directly regulated by this mechanism.

By way of example, test cells are used which carry a regulatory element of a c-Jun target gene fused to a reporter gene whose expression can be easily measured. In the case of fasL, the promotor region including the AP1 binding site, as described *inter alia* by (22), is known and easily available for the person skilled in the art. Alternatively, regulatory sequences of other c-Jun target genes may be employed. Such target genes can be identified by subjecting cells, e.g. primary neurons, derived from junAA mice and control mice, to methods known in the art for determining differential gene and protein expression, e.g. differential display RT-PCR analysis (30,31), DNA microarray technology (12,14,23,29), subtractive hybridization (32,33), or proteome analysis (39,40). Upon identification of the genes, the regulatory sequences and the AP1 binding sites may be obtained by conventional promotor analysis, which involves mutagenesis of the AP1 consensus binding sequences and measuring AP1 dependent reporter gene activation.

An example for conducting a screening assay based on this principle is the following:

In a first step, suitable test cells are selected, i.e. cells which can be easily grown in tissue culture and in which the activation of a reporter gene is dependent on the JNK signaling pathway and c-Jun N-terminal phosphorylation, can be measured. In a preferred embodiment, fibroblasts, which have been extensively used to study the JNK signalling pathway, are used as test cells. Alternatively, neuronal-like cells from established cell lines, like PC-12 (ATCC CRL-1721) cells, may be employed.

The test cells are wild type cells stably transfected, by conventional techniques (38), with a recombinant DNA molecule containing the regulatory region of a target gene for c-Jun N-terminal phosphorylation, e.g. fasL, fused to a reporter gene according to known methods.

Any reporter gene may be used whose expression product is detectable by measuring a signal proportional to its concentration and which can be detected and quantified. Preferably, a reporter gene is employed whose expression can be measured with high sensitivity in an automated assay.

Examples for preferred reporter genes are the luciferase gene (37), the Green Fluorescent Protein GFP (41), and the lacZ gene.

The cells are grown in a suitable medium in microtiter assay plates and subjected to the apoptotic stimulus in the presence or absence of the substances to be tested. Apoptotic stimulation results in reporter gene activity which is expected to be downregulated by substances which exhibit the desired effect, i.e. the ability to inhibit c-Jun N-terminal phosphorylation.

To determine the assay conditions, immortalized cells from control and from junAA mice are tested for differences in apoptosis in response to a variety of stimuli. For example, fibroblasts from both wild type and junAA animals (homozygous for a mutated c-jun allele which has both serines 63 and 73, the JNK phosphoacceptor sites, mutated to alanines) are immortalized according to known procedures and subjected to apoptotic stimuli like UV, ceramide, heat shock, osmotic shock, and other stimuli known to result in c-Jun N-terminal phosphorylation.

Once a suitable stimulus has been identified,which results in reporter gene activity dependent on c-Jun N-terminal phosphorylation, or on signalling pathways resulting in c-Jun N-terminal phosphorylation, the concentration/intensity of the stimulus is determined by varying the concentration/intensitity of the stimulus such that reporter activity is induced in the absence of apoptosis. Alternatively, apoptosis can be blocked by overexpression of an anti-apoptotic gene, e.g. bcl-2.

The screen is conducted by incubating test cells with the test substances in the presence or absence of the apoptotic stimulus, and determining their effect on reporter gene activity.

To confirm the specificity of reporter gene activation for c-Jun N-terminal phosphorylation, or a signalling event upstream, control cells are stably transfected with a recombinant DNA molecule containing a mutated regulatory region of a target gene (preferably the same one as in the in test cell) for c-Jun N-terminal phosphorylation, e.g. fasL, fused to a reporter gene. The mutation in the AP1 binding site is such that AP1 cannot bind and thus AP1 dependent reporter gene activity is abolished. In these control cells, the apoptotic stimulus should not elicit a reporter signal.

Another specificity control is ensured by employing cells transfected with a construct containing a reporter gene fused to the regulatory elements of other c-Jun target genes, which can be identified as described above. By employing various such test cells in parallel, it can be confirmed that the substance has an inhibiting effect on c-Jun N-terminal phosphorylation which results in the downregulation or inhibition of the transcription of c-Jun target genes involved in excitotoxic apoptosis.

To verify that the inhibiting effect of the substance identified is due to inhibition of c-Jun N-terminal phosphorylation (and not to another effect, e.g. an allosteric effect on c-Jun), the test cells may be examined for expression of activated c-Jun upon incubation with the test substance by preparing cell lysates and testing for c-Jun N-terminal phosphorylation, e.g. with antibodies specific for the phosphorylation site, as described (e.g. 10). In order to determine whether the inhibitory effect of the substance on c-Jun N-terminal phosphorylation is due to the inhibition of a signal transduction molecule upstream c-Jun N-terminal phosphorylation, e.g. a JNK or a JNKK (JNK kinase), controls are conducted which allow to determine the site of the inhibitory effect of the compound, e.g. by employing antibodies specific for upstream phosphorylation sites or reporter systems responsive to upstream signalling elements.

In an embodiment of the invention, the above-described cellular assay and the cell-free assay are carried out sequentially or in parallel, thus serving as controls for one another. It can be particularly advantageous to test a substance, which has shown an inhibitory effect on reporter gene activity in the cellular assay, in the cell-tree assay to determine whether this effect is due to c-Jun N-terminal phosphorylation or to an upstream event in the signalling cascade.

Substances that have been identified in the above-described primary screen due to bringing about a decrease in reporter gene activity, are subsequently subjected to a secondary screen in which their specificity for c-Jun N-terminal phosphorylation inhibition, or on a signalling event upstream, is confirmed by ruling out non-specific cytotoxic effects. A suitable, nonradioactive cytotoxicity assay that may be employed in the secondary screen is the LDH (lactate dehydrogenase)release assay (34-36). LDH release assays are commercially available (e.g. Cytotox 96, Promega, Madison, Wisconsin, USA). Alternatively, a propidium iodide cytotoxicity assay (36) may be employed.

In view to the application of the test substances as human drugs, in order to confirm the anti-apoptotic effect of the substance and to determine undesirable side effects in human cells, primary human cells, e.g. neuronal cells, are treated with the substance in the presence or absence of apoptotic stimulation, kainate treatment. This assay will allow for confirming the anti-apoptotic effect of the substance in human cells and its specificity for a given cell type, in the case of neuronal cell death the anti-excitotoxic effect of the substance.

In a further embodiment of the invention, transgenic animals, preferably mice, are provided which are homozygous for a mutated c-jun allele which has both serines 63 and 73, the JNK phosphoacceptor sites, mutated, preferably to alanines (junAA animals).

These mice are obtainable by standard ES-cell technology by injecting blastocysts with ES-cells in which the endogenous c-jun gene was replaced by the mutant c-jun allele. For obtaining such knock-in transgenic animals conventional techniques, e.g. the CRE/loxP system, as described by (13, 43), may be employed.

The JunAA mice of the invention can be used to identify target genes regulated by c-Jun N-terminal phosphorylation during apoptosis and other biological processes. In a first step, primary cells, e.g. neurons or T-cells, from junAA and from wildtype control mice are isolated. Next, the cells are subjected to a apoptotic stimulus, like kainate, as described above for the screen, and differences in RNA or protein levels are determined by standard methods for differential gene transcription and/or protein expression, as described above.

It has been shown in experiments of the present invention that fibroblasts lacking JNP have a proliferation defect, but grow faster than *c-jun*-/-cells. Similarly, *junAA* mice are smaller than controls, but ubiquitous deletion of a floxed *c-jun* allele using the cre-expressing Ball transgenic line (24) results in a much more dramatic growth retardation. Therefore JNP seems to be essential for some, but not all functions of c-Jun in these two processes. In contrast, JNP is not required for the apparent anti-apoptotic function of c-Jun during hepatogenesis, but c-Jun appears to be the essential substrate of JNK3 for the epileptogenic effects and the excitotoxic neuronal damage induced by kainate. Likewise, in the absence of c-Fos, fibroblasts are hypersensitive to UV-induced death, but c-Fos-deficient photoreceptor neurons are resistant to light-induced apoptosis (25). Therefore AP-1 transcription factors and JNP seem to have both protective and inductive functions during apoptosis.

The present invention provides the first genetic evidence for the regulation of c-Jun by the JNK signaling pathway *in vivo* and for the role of JNP as a molecular switch, which increases the spectrum of c-Jun's functions, possibly by regulating the recruitment of distinct coactivator complexes.

Since c-Jun N-terminal phosphorylation does not seem to be required for vital physiological functions, drugs that inhibit c-Jun N-terminal phosphorylation may be used to treat diseases caused by excitotoxic apoptosis, e.g. glutamate neurotoxicity, which has been associated with a number of clinically relevant neurologic diseases including epilepsy, stroke and Huntington's disease (26).

### Brief description of the Figures

Fig. 1
   A) Targeting strategy to obtain ES-cells carrying a junAA allele
   B) Genotype analysis of wildtype and junAA mice by PCR
   C) Sequence analysis of the c-Jun N-terminus in wild type and junAA mice
   D) Postnatal growth rate of wild-type and *junAA* homozygous mice
Fig. 2
   A) Proliferation rates of wild type and junAA mouse embryonic fibroblasts
   B) Expression and UV-induction of *c-jun*, *c-fos* and *fox* mRNA in wild type and junAA mouse embryonic fibroblasts
   C) AP1 activity in wild type and junAA mouse embryonic fibroblasts
   D) Western blot analysis of wild-type and *junAA* primary fibroblasts following UV-irradiation
Fig. 3
   A) Kainate-induced seizures in adult wild-type and *junAA* homozygous mice
   B) Histological analysis and TUNEL staining of wild-type and *junAA*/*junAA* hippocampi and cortices
Fig. 4
   A) Kainate-induced neuronal cell death in vitro
   B) Immunofluorescence analysis of wild-type and *junAA* neurons
   C) JNK in-gel kinase assay of untreated or kainate-treated
   D) RT-PCR analysis of *fasL* expression in wild-type and *junAA* neuronal cultures without and after addition of kainate

### Example 1

### Generation of transgenic mice lacking c-Jun N-terminal phosphorylation

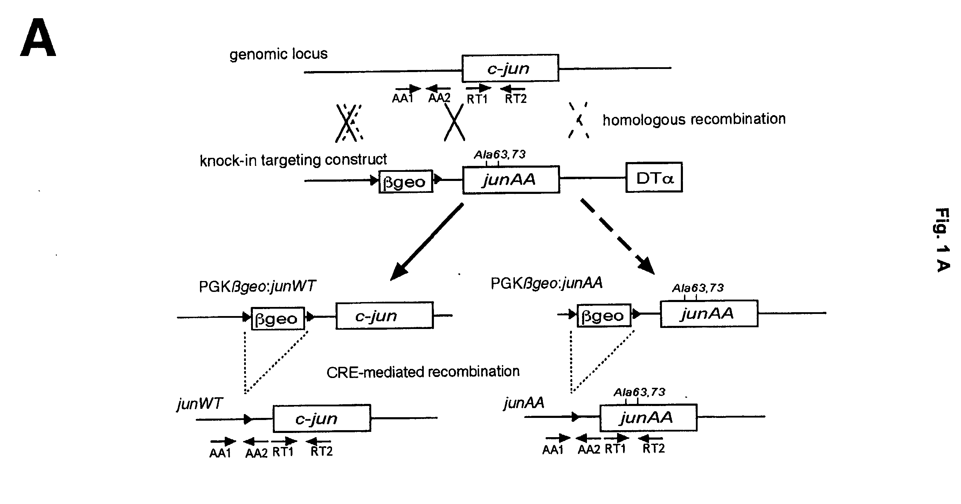

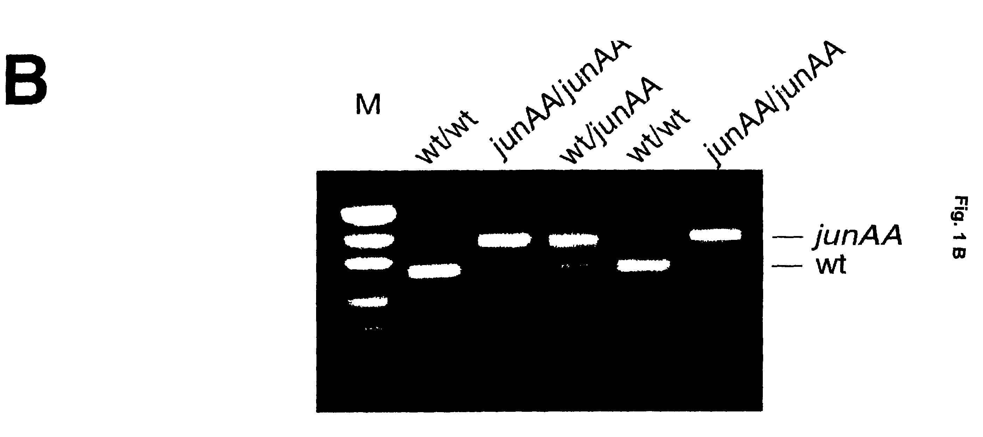

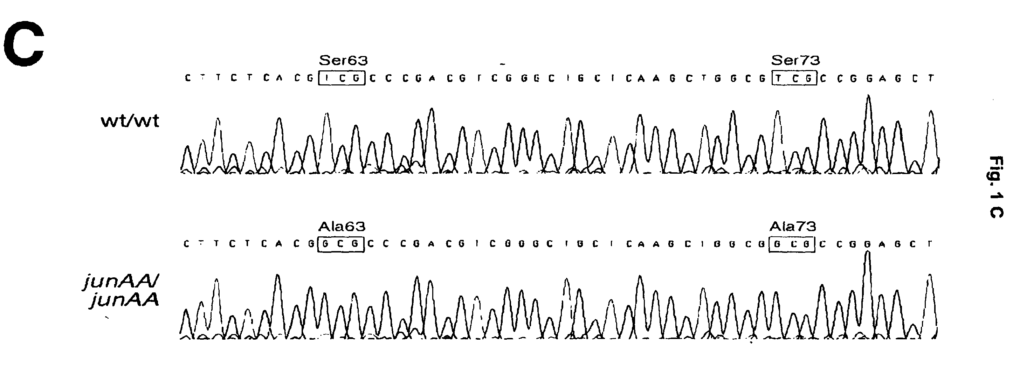

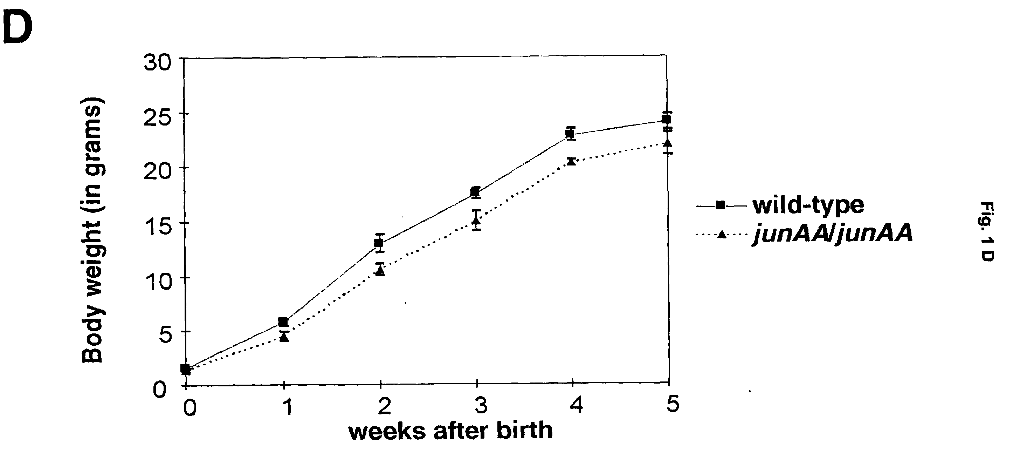

To examine the role of JNP in vivo, mice harboring a mutant allele of *c-jun*, which has both serines 63 and 73 mutated to alanines (*junAA*) as well as mice harboring a control wild-type allele (*junWT*) (13) (Fig. 1A-C) were generated.

For the knock-in targeting vector, a 5.5-kb Sac1-Xba1 genomic fragment encompassing the 5' untranslated region, the *c-jun* coding sequence and 3.5-kb 3' region were cloned into Sac1 and Xba1 sites of pBluescript. The serine codons 63 and 73 were changed to alanines by site-directed PCR-based mutagenesis. A Sal1 linker was inserted into the EcoR1 site present at position -360 relative to the ATG, and the floxed PGKβgeo selection marker inserted as a Sal1-Xho1 fragment. The DTα gene was cloned as a Pst1-Not1 fragment into the Nsi1-Not1 sites of the vector.

20µg of the Not1-linearized knock-in construct were electroporated into R1 ES cells (28). Selection and screening of ES cell colonies was performed with two sets of primers as described (28), and the correct targeting was confirmed by Southern blot analysis. Out of 282 colonies analyzed, 14 homologous recombinants had cointegrated the point mutations (PGKβ*geo*:*junAA*) and 1 had not (PGKβ*geo*:*junWT*). Chimeric mice were generated by microinjection of individual ES cells clones carrying the PGKβgeo:*junWT* or the PGKβgeo:*junAA* allele into C57BL/6 blastocysts. Several chimeras transmitted the respective mutant allele to their offspring. The floxed PGKβgeo gene was removed from the germline of PGKβ*geo*:*junAA* and PGKβ*geo*:*junWT* mice by pronuclear injection of a cre-expressing plasmid as described in (13).

*junWT*/*junWT* and *junWT*/- mice are indistinguishable from wild-type indicating that the loxP site present in the promoter does not affect the biological activity of the locus. Surprisingly, *junAA* homozygotes were obtained at Mendelian frequency. At birth, *junAA* homozygotes were indistinguishable from wild-type littermates. However, a small but significant difference in body weight was observed starting from the first week after birth, which persisted, to adulthood (Fig. 1D). Adult *junAA* homozygotes and *junAA*/- mice of both sexes were healthy and fertile and histological examination of several organs including the liver revealed no obvious abnormalities. Therefore, c-Jun is not an essential target for SEK1 signaling during hepatogenesis, although c-Jun seems to have a function independent of the SEK/JNK pathway in this biological process (6).

Fig. 1. (A) depicts the schematic representation of the targeting strategy used to knock-in a mutant *c-jun* allele, which has the JNK phosphoacceptor serines 63 and 73 mutated to alanines into the *c-jun* locus. Homologous recombination between the solid or dashed lines generates the mutant PGKβ*geo*:*junAA* or the wild-type PGKβ*geo*:*junWT* alleles, respectively. The *junAA* and the *junWT* alleles were obtained after removal of the PGKβ*geo* cassette, which is flanked by LoxP sites represented by filled triangles. (B) Genotype analysis of progeny from a heterozygous (*junAA*/+) intercross. PCR analysis of tail DNA isolated from 3-week old mice was performed using the PCR primers AA1 and AA2 shown in (A). The genotype is indicated above, the expected wild-type (wt) and mutant bands on the side. M, DNA marker ΦX174 cut with HaeIII. (C) Nucleotide sequence determination of the *c-jun* N-terminus of wild-type and *junAA* homozygotes. PCR fragments encompassing the *c-jun* N-terminus were amplified from tail DNA using the primers RT1 and RT2 shown in (A). Sequencing of both DNA strands was performed using RT1 and RT2 as sequencing primers. Only the sequence of the coding strand is shown. (D) Postnatal growth rate of wild-type and *junAA* homozygous mice. Body weights are shown with postnatal age. Eight female mice per group were scored, and each stage represents the mean value +/- S.E.M.. Similar results were obtained when the growth rate of male mice was compared.

### Example 2

### c-Jun N-terminal phosphorylation is required for efficient fibroblast proliferation

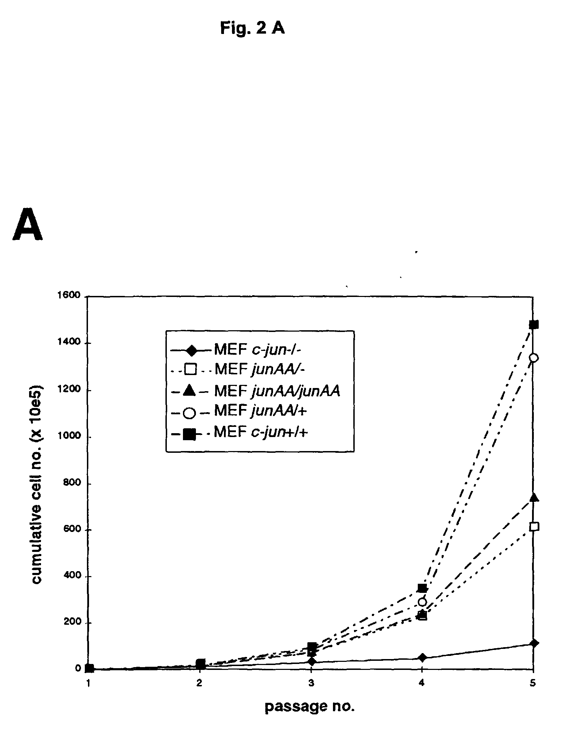

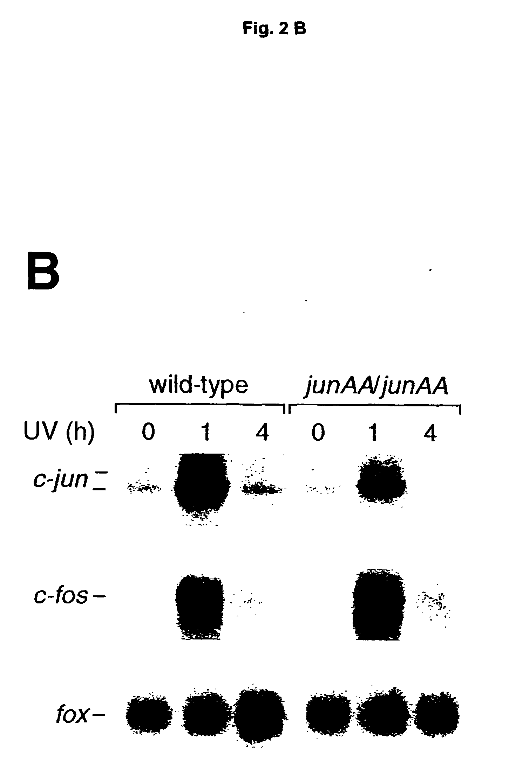

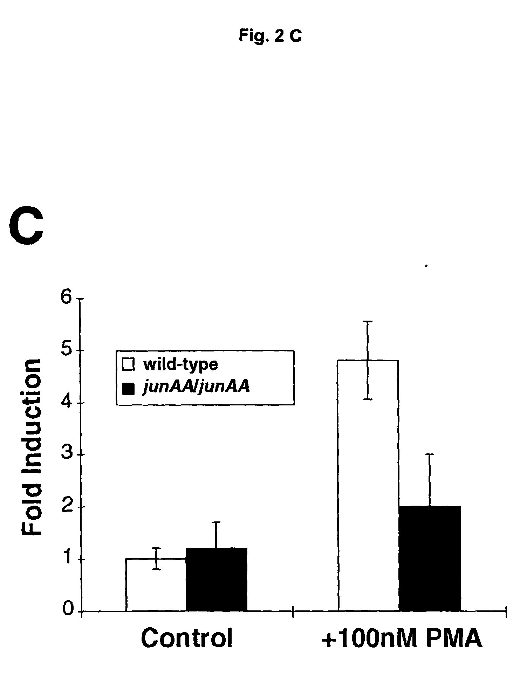

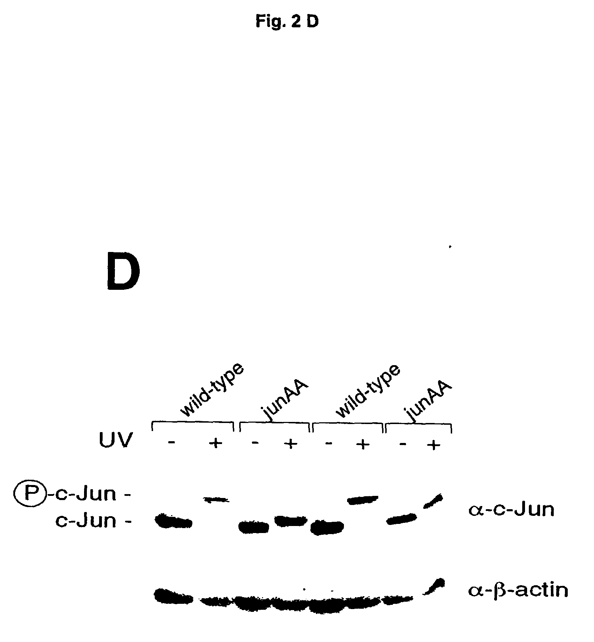

To examine whether JNP was required for cell proliferation in vitro, primary fibroblasts of various genotypes were isolated from E12.5 embryos and their growth rate determined. *junAA*/*junAA* and *junAA*/-fibroblasts had a proliferation defect but did not enter premature senescence, as did *c-jun*-/- cells (Fig. 2A). The slower proliferation rate of *junAA* fibroblasts was not due to increased cell death, since the levels of background apoptosis were comparable between wild-type and mutant cultures. The *c-jun* gene itself, which is autoregulated by c-Jun (15), could be a possible target gene responsible for the proliferation defect of *junAA* fibroblasts. In mutant fibroblasts basal *c-jun* mRNA levels were comparable to wild-type, whereas the induction of *c-jun* transcription by UV irradiation was reduced by 35% (Fig. 2B). Likewise, stimulation of AP-1 transcriptional activity by PMA was decreased in mutant fibroblasts (Fig. 2C). The JNK pathway through phosphorylation of the ternary factor protein ELK1 (16) regulates UV induction of the *c-fos* gene. However, basal transcription and UV-inducibility of *c-fos* were not affected by lack of JNP (Fig. 2B). Previous findings that serines 63 and 73 are the main targets of JNK phosphorylation on the c-Jun protein (17) were confirmed by Western analysis (Fig. 2D). Stimulation of JNK activity by UV irradiation of wild-type primary fibroblasts resulted in the appearance of a slow migrating phosphorylated form of c-Jun, but mutation of serines 63 and 73 almost completely abolished the change in electrophoretic mobility (Fig. 2D). The residual UV-induced modification of c-Jun is likely to be due to phosphorylation at threonines 91, 93 and 95 by an unidentified kinase activity (17).

The results of these experiments are shown in Fig. 2. (A) Proliferation rates of mouse embryonic fibroblasts (MEFs). Primary cells were isolated, counted and passaged at 3-day intervals and the cumulative cell number calculated. (B) Expression and UV-induction of *c-jun*, *c-fos* and *fox* mRNA (loading control) in MEFs (passage 3). Total RNA was isolated and Northern analysis was performed as described (27). (C) Primary fibroblasts were cotransfected with a luciferase reporter gene regulated by a pentameric AP-1 site (5x TRE-Luc) and CMV-LacZ (transfection control). Cell extracts were harvested 16 hours after PMA treatment. Each value represents the mean +/- S.E.M. of two independent primary clones of each genotype measured in duplicates. (D) Western blot analysis of two independent wild-type and *junAA* primary fibroblast clones following UV-irradiation (100J, 45 min., indicated by +) or mock treatment (-). Western blot analysis was performed as described in (28). c-Jun-specific (Transduction Laboratories) and β-actin-specific (Sigma, loading control) antibodies were used for immunodetection.

### Example 3

### c-Jun is the essential substrate of JNK-mediated excitotoxic neuronal death

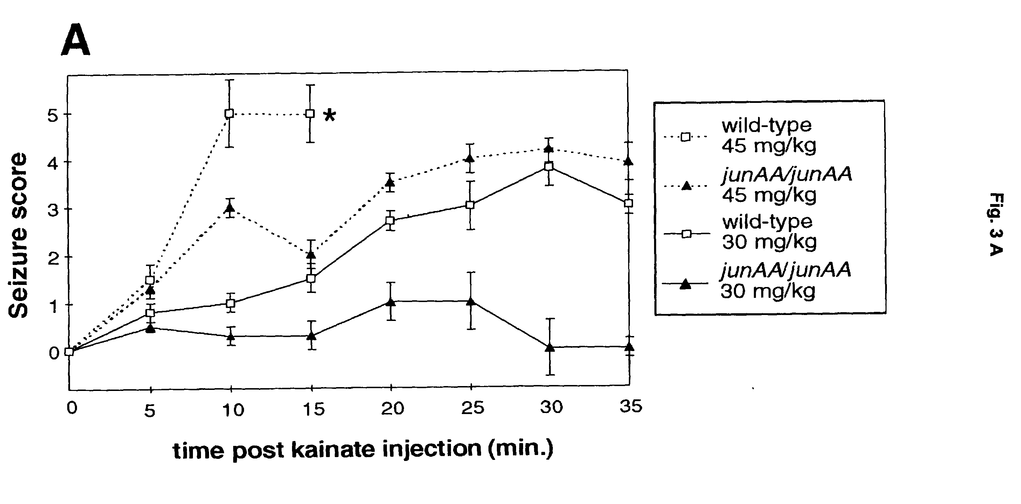

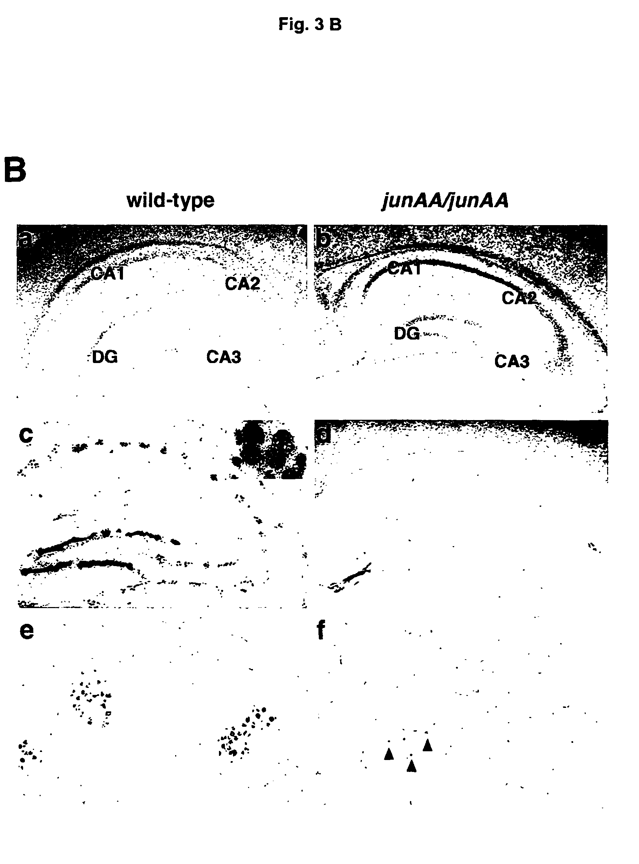

Glutamate-receptor agonists activate the JNK pathway in cultured neurons (18) and JNP correlates with kainate-induced neuronal damage in vivo (10). Kainate elicits epileptic seizures by direct stimulation of the AMPA/kainate class of glutamate receptors and indirectly by increasing the release of excitatory amino acids from nerve terminals (19). In addition, systemic administration of kainate induces a well-defined pattern of neuronal degeneration in the hippocampus and in other brain regions (20). Whereas administration of kainate to wild-type mice (Fig. 3A) and *junWT* homozygotes induced severe seizures which lasted for 1-2 hours, *junAA*/*junAA* mice showed milder symptoms and recovered much faster (Fig. 3A). Barrel rotations, which are judged as a particular severe form of motor seizures, were observed in controls receiving 30mg/kg (2/12) and 45mg/kg kainate (7/12), but were absent in mice lacking JNP (0/12). Moreover, at the high dose (45mg/kg kainate) 6/12 wild-type mice died during epilepsy, whereas no mortality was observed in *junAA*/*junAA* mice (0/12). At the histological level, the brains of *junAA* mice were indistinguishable from wild-type (Fig. 3B) indicating that differences in the neuroanatomy or cellular composition are not the cause for the altered kainate sensitivity of mutant mice. Five days after kainate treatment (30mg/kg) 5/6 wild-type mice showed increased apoptosis in the hippocampus as revealed by TUNEL staining (Fig. 3B). Consistent with previously published studies (10, 20), the CA1 (4/6) and the CA3 (3/6) subfields were the most vulnerable hippocampal regions, although apoptosis could also be detected in CA2 neurons (2/6) and in the dentate gyrus (1/6) (Fig. 3B). Dying wild-type neurons showed morphological characteristics of apoptosis like membrane blebbing and nuclear condensation, but no apoptosis was detectable in the hippocampi of *junAA* mice (0/6) (Fig. 3B). 3/6 control animals showed clusters of TUNEL-positive cells in the cortex whereas in only 1/6 mutant mice a small number of dying neurons could be detected (Fig. 3B).

Fig. 3. (A) shows the the kainate-induced seizures in adult wild-type and *junAA* homozygous mice. Behavioral alterations induced by kainate treatment were scored according to (10): 1, arrest of motion; 2, myoclonic jerks of the head and neck, with brief twitching moments; 3, unilateral clonic activity; 4, bilateral forelimb clonic and tonic activity; 5, generalized tonic-clonic activity with loss of posture and death during epilepsy. Eight mice per group were analyzed and the mean values are shown +/- S.E.M.. The asterix indicates that 3/12 wild-type mice were dead after 15 min., 3 more mice died within one hour after kainate injection. (B) Histological analysis and TUNEL staining of wild-type and *junAA*/*junAA* hippocampi and cortices. Nissl staining of wild-type (a) and *junAA*/*junAA* (b) hippocampi. TUNEL labeling showing apoptotic neurons in the dentate gyrus (DG) and the CA1-CA3 subfields in wild-type (c), not in mutant hippocampi (d) and in wild-type (e) and *junAA*/*junAA* (f) cortices. Black arrowheads indicate some apoptotic nuclei in (f). The inset in (c) shows a high magnification of TUNEL-stained CA1 neurons. TUNEL labeling was performed as described in (28).

### Example 4

### c-Jun N-terminal phosphorylation is required for kainate-induced neuronal apoptosis in vitro

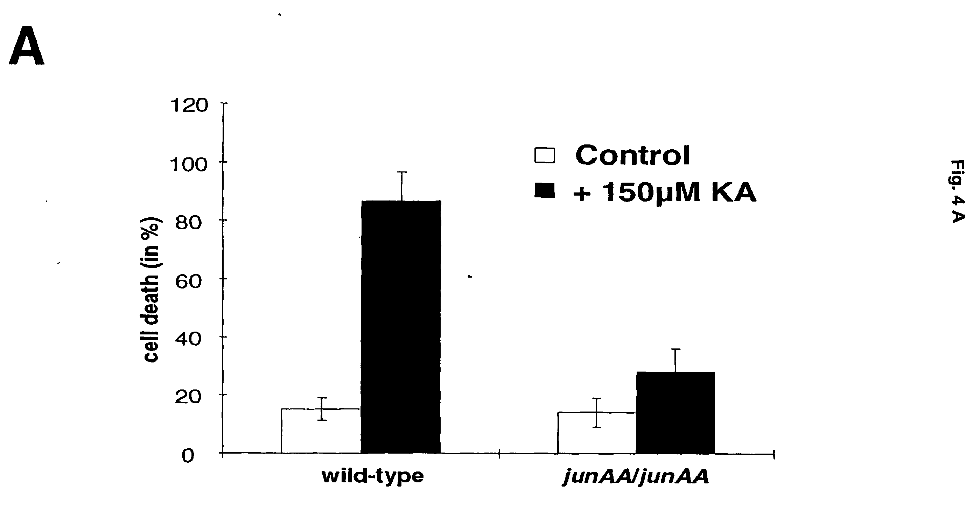

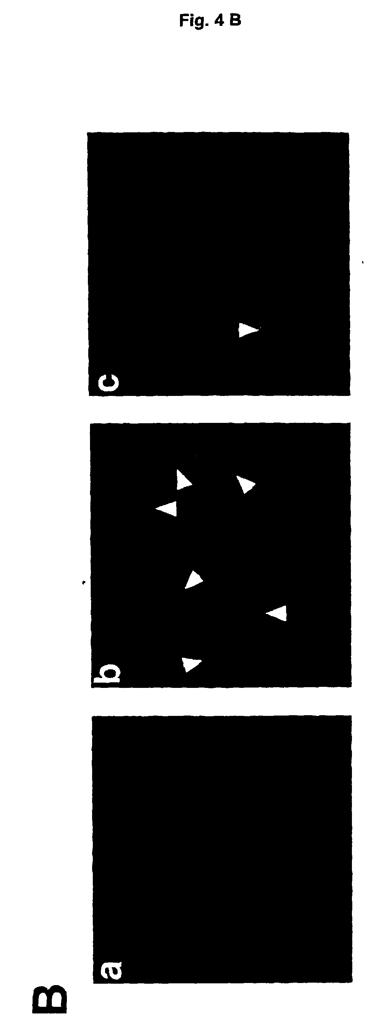

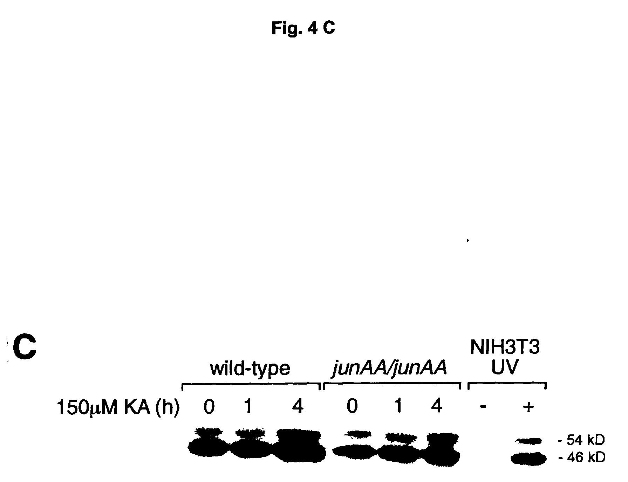

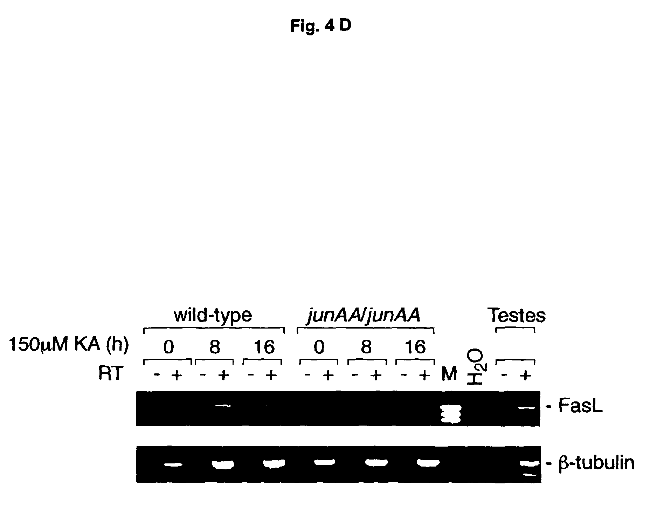

To analyze the mechanism of c-Jun-mediated excitotoxicity, purified primary cortical /hippocampal neurons isolated from wild-type and *junAA* mice were tested for kainate-sensitivity in vitro (21) (Fig. 4). Administration of 150µM kainate induced cell death in 85% of control neurons after 24 hours, but only 23% of mutant neurons died suggesting a cell-autonomous requirement for JNP (Fig. 4A). Both the basal and the kainate-induced JNK activity were unchanged in *junAA* neurons (Fig. 4C) indicating that the signaling pathways connecting the kainate receptors with the JNKs are intact in mutant neurons. Although *junAA* neurons were less susceptible to excitotoxic effects of kainate, a few mutant cells died also by apoptosis (Fig. 4B).

JNP might be required for the efficient transcription of target genes necessary to execute neuronal apoptosis. The gene encoding the apoptosis inducer FasL has been shown to be regulated by AP-1 in T-cells and JNP correlates with induced expression of *fasL* after cerebral ischemia-reperfusion (22). To test whether *fasL* was regulated by JNP during excitotoxicity, the mRNA levels of *fasL* were determined by RT-PCR 8 and 16 hours after kainate treatment. RNA preparation and reverse transcription were performed as described in (22). The primers used for PCR amplification were: 5'- CAG CAG TGC CAC TTC ACT TTG G-3' and 5'- TTC ACT CCA GAG ATC AGA GCG G-3' for fasL and 5'-CTT CTG CTC CTC ACA GAC CTC-3' and 5'-GAC AGA GGC AAA CTG AGC ACC-3' for β-tubulin.

Kainate stimulation of wild-type neurons resulted in the transcriptional induction of the *fasL* gene after 8 hours to levels present in testes, a known site of *fasL* expression (22) (Fig. 4D). However, only very low levels of *fasL* mRNA could be detected in mutant neurons after 16 hours in response to kainate (Fig. 4D). Therefore, *fasL* is probably one important target gene regulated by JNP during neuronal apoptosis.

Fig. 4. (A) Kainate-induced neuronal cell death in vitro. At day 4 in vitro, kainate (KA, final concentration 150µM) was added to the culture medium. The presence of live or dead neurons was evaluated after 24h. The percentage of cell death +/- S.E.M. in control-treated (white bars) and kainate-treated (black bars) cultures is shown, the genotypes are indicated below. Neuronal cultures and apoptosis assays were performed as described in (21). (B) Immunofluorescence analysis of wild-type and *junAA* neurons. Immunostaining for the neuronal marker MAP2 is shown in red, apoptotic cells are labeled in green (29). Wild-type (a, b) and mutant (c) primary neurons with (b, c) and without (a) kainate-treatment are shown. White arrowheads indicate apoptotic nuclei. (C) JNK in-gel Kinase assay of untreated or kainate-treated (150µM) wild-type and *junAA* primary neurons. JNK activity in neuronal lysates (25µg) and NIH3T3 lysates (10µg) was measured using the substrate glutathione S-transferase (GST)-c-Jun as described (22). The molecular weight of the JNK isoenzymes is indicated on the side, the genotypes and the time after kainate-treatment on top. Mock-treated (-) and UV-irradiated (100J, 30 min., indicated by +) NIH3T3 fibroblasts were used as controls.

(D) RT-PCR analysis of *fasL* expression in wild-type and *junAA* neuronal cultures without and after addition of kainate (150µM). The PCR fragments detecting FasL and β-tubulin (loading control) expressions are indicated on the side, the genotypes and the time after kainate-treatment on top. cDNA from testes was used as a positive control. Each sample was analyzed with (RT+) and without (RT-) reverse transcription. M, DNA marker ΦX174 HaeIII; H₂O, no cDNA added.

Neuronal apoptosis was detected using the In Situ cell Death Detection Kit (Boehringer Mannheim). MAP-2 immunofluorescence staining was performed using a monoclonal anti-MAP-2 antibody (Sigma, 1:200) as the first, and CY3-linked mouse immunoglobulins (Jackson Laboratories, 1:300) as secondary antibodies.

## Claims

1. Method for identifying substances for the treatment of disorders associated with c-Jun-mediated apoptosis, characterized in that substances are tested for their ability to inhibit c-Jun N-terminal phosphorylation.

2. The method of claim 1, characterized in that the substances are tested for their ability to inhibit the physical interaction of a c-Jun N-terminal kinase, preferably JNK3, with its substrate, c-Jun.

3. The method of claim 2, characterized in that the JNK binding domain on c-Jun and the c-Jun binding domain on JNK, or proteins containing said domains, one of them being fused to a reporter gene product and the other one being immobilized on a carrier, are incubated with the test substance and the effect of the substance on the physical interaction of the two proteins is measured by measuring the reporter activity.

4. The method of claim 3, characterized in that the JNK is JNK3.

5. The method of claim 3, characterized in that the reporter gene is Green Fluorescent Protein.

6. The method of claim 1, wherein mammalian cells transfected with a reporter gene construct are subjected to an apoptotic stimulus, incubated with a test substance, and the reporter gene activity is measured, characterized in that the activation of the reporter gene is dependent on c-Jun N-terminal phosphorylation, or a signal transduction event upstream of c-Jun N-terminal phosphorylation, and that the apoptotic stimulus is defined by having a different effect on cells derived from wild-type animals and on cells derived from animals which are homozygous for a mutated c-jun allele which has the JNK phosphoacceptor sites mutated.

7. The method of claim 6, characterized in that both serines 63 and 73 are mutated to alanines.

8. The method of claim 6, characterized in that the cells are transfected with a reporter gene which is under the control of a regulatory element of a c-Jun target gene.

9. The method of claim 7, characterized in that the c-Jun target gene is fasL.

10. Substances identified in a method of one of the claims 1 - 10 for the treatment of disorders causes by excitotix apoptosis.

11. The substances of claim 11 for the treatment of neurodegenerative diseases.

12. Transgenic animal, characterized in that it is homozygous for a mutated c-jun allele which has the JNK phosphoacceptor sites mutated.

13. The transgenic animal of claim 10, characterized in that both serines 63 and 73 are mutated to alanines.

14. Cells derived from the transgenic animal of claim 10 or 11.
